(19) Europäisches Patentamt
European Patent Office
Office européen des brevets

(11) Veröffentlichungsnummer: **0 319 814 B1**

(12) **EUROPÄISCHE PATENTSCHRIFT**

(45) Veröffentlichungstag der Patentschrift: **16.09.92**

(51) Int. Cl.$^5$: **C07D 211/90**

(21) Anmeldenummer: **88119772.7**

(22) Anmeldetag: **28.11.88**

# (54) Verfahren zur Herstellung von unsymmetrischen Dihydropyridinen.

(30) Priorität: **08.12.87 DE 3741540**

(43) Veröffentlichungstag der Anmeldung:
**14.06.89 Patentblatt 89/24**

(45) Bekanntmachung des Hinweises auf die Patenterteilung:
**16.09.92 Patentblatt 92/38**

(84) Benannte Vertragsstaaten:
**AT BE CH DE ES FR GB GR IT LI NL SE**

(56) Entgegenhaltungen:
**EP-A- 0 095 450**
**EP-A- 0 124 743**
**DE-A- 2 117 571**
**DE-A- 2 117 573**

(73) Patentinhaber: **BAYER AG**
**W-5090 Leverkusen 1 Bayerwerk(DE)**

(72) Erfinder: **Naab, Paul, Dr.**
**Amalienstrasse 29**
**W-5600 Wuppertal 2(DE)**
Erfinder: **Lange, Willi**
**Gellertweg 32**
**W-5600 Wuppertal 1(DE)**
Erfinder: **Teller, Werner, Dr.**
**In den Birken 79**
**W-5600 Wuppertal 1(DE)**

EP 0 319 814 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein chemisch eigenartiges Verfahren zur Herstellung von unsymmetrischen 1,4-Dihydropyridincarbonsäureestern.

Aus der deutschen Offenlegungsschrift 2 117 571 ist die Verbindung 4-(3-Nitrophenyl)-2,6-dimethyl-1,4-dihydropyridin-3,5-carbonsäure-methyl-ethylester (nachfolgend Nitrendipin genannt) bereits bekannt ebenso wie Verfahren zu ihrer Herstellung (vgl. auch DE-OS 2 117 573). In EP-A-01 24 743 wird ebenfalls die Herstellung von unsymmetrischen Dihydropyridinen durch Umsetzung von Ylidenverbindungen mit Enaminocarbonsäureestern beschrieben. In der EP-Anmeldung 0 124 743 werden die einzusetzenden Ylidenverbindungen durch die Verwendung bestimmter Katalysatoren in besonderer Reinheit und Ausbeute erhalten, was bei der weiteren Umsetzung zu Dihydropyridinen erneut zu sehr reinen Endprodukten führen soll.

Nitrendipin ist ein anerkannter Arzneiwirkstoff der bereits in zahlreichen Ländern arzneimittelrechtlich zugelassen ist und medizinisch angewendet wird. Für solche Arzneiwirkstoffe besteht das Bedürfnis nach besonderer Reinheit. Bei der Herstellung von unsymmetrischen Dihydropyridinen, insbesondere auch bei der Herstellung von Nitrendipin, entstehen neben dem gewünschten Wirkstoff bisher auch immer symmetrische Ester in beträchtlichem Umfang, die als unerwünschte Nebenprodukte nur mühsam und aufwendig von den entsprechenden unsymmetrischen Estern abgetrennt werden können (vgl. folgendes Formelschema).

Nitrendipin

(unsymmetrischer Ester)

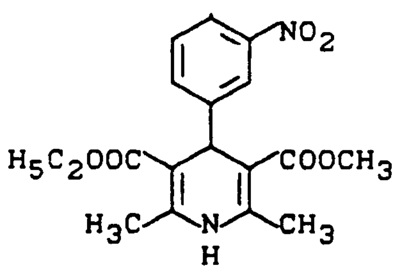

I

Symmetrische Ester

(unerwünschte Nebenprodukte)

A

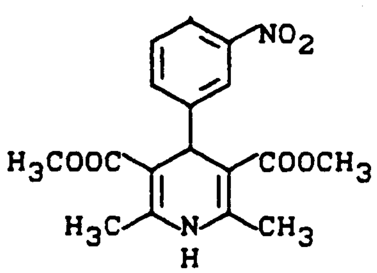

B

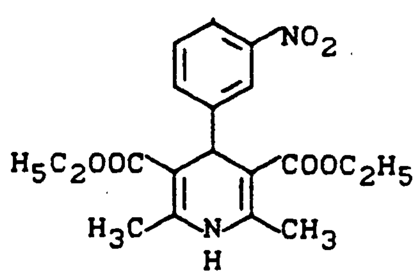

Die Erfindung betrifft ein Verfahren zur Herstellung von Nitrendipin durch Umsetzung einer Ylidenverbindung der allgemeinen Formel II (IIa) bzw. (IIb)

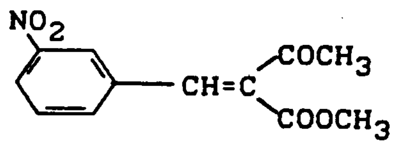

(IIa)

II

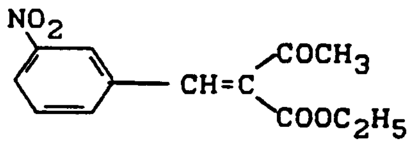

(IIb)

mit einer Enaminverbindung der allgemeinen Formel III (IIIa) bzw. (IIIb)

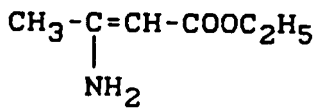

(IIIa)

III

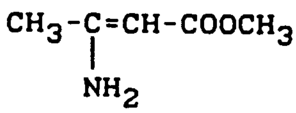

(IIIb)

dadurch gekennzeichnet, daß man die Reaktion in einem organischen Lösungsmittel in Gegenwart von katalytischen Mengen von Diisopropylaminacetat oder Dimethylbenzylaminacetat durchführt.

Als organische Lösungsmittel kommen vorzugsweise aliphatische Alkohole mit 1 bis 6 C-Atomen, insbesondere Methanol, Ethanol und/oder Isopropanol zur Anwendung.

Die Umsetzung wird vorzugsweise bei Temperaturen zwischen -10 und 150° C, insbesondere zwischen 40 und 100° C, durchgeführt.

Der Katalysator wird bevorzugt in Mengen von 0,001 bis 0,009 Mol, insbesondere von 0,003 bis 0,008 Mol, pro Mol Ylidenverbindung, eingesetzt.

Es wurde überraschenderweise gefunden, daß der Ringschluß aus Ylidenverbindung und Enaminen durch die Verwendung der erfindungsgemäßen Katalysatoren, insbesondere bei Beachtung der genannten Konzentrationsangaben, in sehr hohen Ausbeuten (mehr als 90 % der Theorie) verläuft und gleichzeitig nur äußerst geringe Mengen von unerwünschten symmetrischen Estern gebildet werden. Darüber hinaus ist die Reaktionszeit im Vergleich zu den bisher bekannten Methoden wesentlich kürzer. Die Umsetzung ist in der Regel bereits nach wenigen Stunden beendet.

Zum Nachweis des unerwarteten Vorteils des erfindungsgemäßen Verfahrens wurde die Herstellung von Nitrendipin gemäß den Ausführungsbeispielen aus EP-A-01 24 743 wiederholt. Durch Kombination der Beispiele 1 und 2 erhält man Nitrendipin in einer Ausbeute von ca. 80 % der Theorie (Fp.: 159° C). Ein dünnschichtchromatographische Untersuchung aus Merck-Kieselgelplaten (Laufmittel: Chloroform: Aceton:Petrolether = 3:2:5) zeigt keine sichtbaren Nebenprodukte.

Eine chromatographische Untersuchung mittels HPLC (z.B. Hibar Fertigsäule, Merck, Darmstadt) (Säule: Länge 12,5 cm, Ø 4 mm, Füllung LiChrosorb RP 18, 5 µm. Elutionsmittel: Acetonitril/Tetrahydrofuran/Wasser = 120/240/640 (V/V). Fluß: 1,5 ml/min. Detektorwellenlänge: 235 nm. Relative Retentionszeiten: Nitrendipin 1,00; Symmetrischer Ester A 0,75, symmetrischer Ester B 1,35.) ergibt jedoch, daß das erhaltene Nitrendipin stark verunreinigt ist durch symmetrische Ester. Von dem Methylester (Formel A) werden 2,64 % und von dem Ethylester (Formel B) werden 2,55 % als Verunreinigung nachgewiesen. Selbst nach nochmaliger Umkristallisation aus Methanol in Gegenwart von Aktivkohle (Kristallisationsausbeute ca. 85 %) werden bei erneuter HPLC-Untersuchung die symmetrischen Nebenprodukte A und B zu 2,27 % und 2,34 % nachgewiesen. Dieser Kristallisationsversuch zeigt gleichzeitig die Schwierigkeit, die symmetrischen Nebenprodukte vom Nitrendipin zu trennen.

Die folgenden Ausführungsbeispiele zeigen beispielhaft die Geschwindigkeit des erfindungsgemäßen

Verfahrens, seine hohen Ausbeuten und den unerwartet geringen Anteil an unerwünschten symmetrischen Verbindungen.

Beispiel 1

45 g (0,39 Mol) Aminocrotonsäuremethylester, 92,5 g (0,35 Mol) 3-Nitrobenzylidenethylester (hergestellt aus Acetessigsäureethylester und 3-Nitrobenzaldehyd), werden mit 2,5 ml einer 10 %igen Lösung von Dimethylbenzylaminacetat (nachfolgend Desmorapidacetat; hergestellt aus entsprechenden Essigsäure und Dimethylbenzylamin in Isopropanol und verdünnt auf 100 ml; ≙ 1,3 mmol) in 275 ml Isopropanol zum Sieden erhitzt und 5 Stunden am Rückfluß gerührt. Dabei werden insgesamt 500 ml Isopropanol abdestilliert und wieder durch frisches Lösungsmittel ergänzt. Der Ansatz wird auf 50° C abgekühlt, mit Nitrendipin angeimpft und über Nacht bei Raumtemperatur gerührt. Danach wird auf 0 - 5° C abgekühlt, 2 Stunden bei dieser Temperatur gerührt, abgesaugt und mit 90 ml Methanol gewaschen und über Nacht bei 40° C im Vakuum getrocknet.

Ausbeute: 121,1 g (≙ 96 % der Theorie)

Nach HPLC lassen sich 0,13 % des symmetrischen Esters A nachweisen. Der Gehalt des symmetrischen Esters B liegt unter 0,05 %.

**Patentansprüche**

1. Verfahren zur Herstellung von Nitrendipin durch Umsetzung einer Ylidenverbindung der allgemeinen Formel II

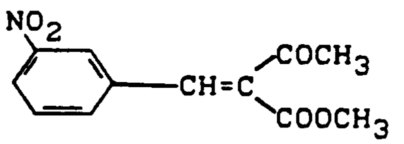

(IIa)

II

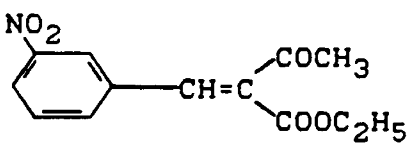

(IIb)

mit einer Enaminverbindung der allgemeinen Formel III (IIIa) bzw. (IIIb)

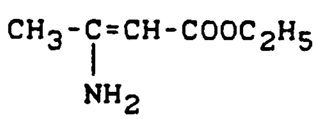

(IIIa)

III

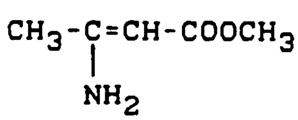

(IIIb),

dadurch gekennzeichnet, daß man die Reaktion in einem organischen Lösungsmittel in Gegenwart von katalytischen Mengen von Diisopropylaminacetat oder Dimethylbenzylaminacetat durchführt.

2. Verfahren gemäß Anspruch 1, dadurch gekennzeichnet, daß man als organische Lösungsmittel aliphatische Alkohole mit bis zu 6 C-Atomen einsetzt.

3. Verfahren gemäß Anspruch 1, dadurch gekennzeichnet, daß man die Umsetzung zwischen -10 und

150° C durchführt.

4. Verfahren gemäß Anspruch 1, dadurch gekennzeichnet, daß man die Umsetzung in Gegenwart von 0,001 bis 0,009 Mol des Katalysators, bezogen auf die molaren Mengen der Ylidenverbindung II, durchführt.

5. Verwendung von Diisopropylaminacetat oder Dimethylbenzylaminacetat als Katalysator bei der Herstellung von Nitrendipin.

## Claims

1. Process for the preparation of nitrendipine by reaction of an ylidene compound of the general formula II

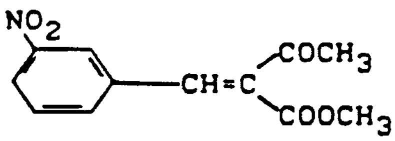
(IIa)

II

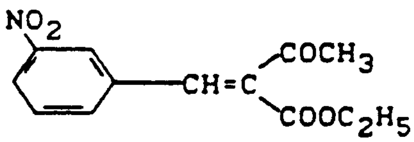
(IIb)

with an enamine compound of the general formula III (IIIa) or (IIIb)

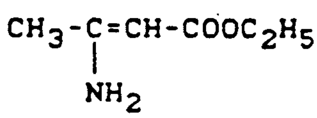
(IIIa)

III

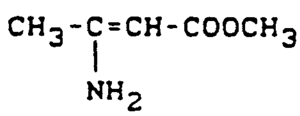
(IIIb),

characterised in that the reaction is carried out in an organic solvent in the presence of catalytic amounts of diisopropylamine acetate or dimethylbenzylamine acetate.

2. Process according to Claim 1, characterised in that aliphatic alcohols having up to 6 carbon atoms are employed as organic solvents.

3. Process according to Claim 1, characterised in that the reaction is carried out between -10 and 150° C.

4. Process according to Claim 1, characterised in that the reaction is carried out in the presence of 0.001 to 0.009 mol of catalyst, relative to the molar amounts of the ylidene compound II.

5. Use of diisopropylamine acetate or dimethylbenzylamine acetate as a catalyst in the preparation of nitrendipine.

## Revendications

1. Procédé de production de nitrendipine par réaction d'un composé ylidénique de formule générale II

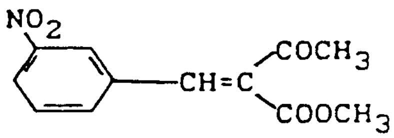 (IIa)

II

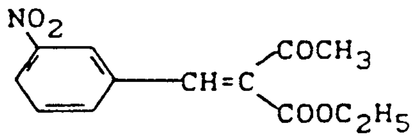 (IIb)

avec un composé d'énamine de formule générale III (IIIa) ou (IIIb)

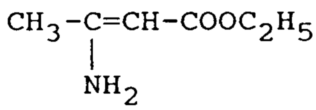 (IIIa)

III

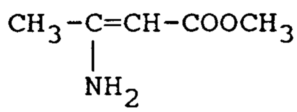 (IIIb),

caractérisé en ce qu'on conduit la réaction dans un solvant organique en présence de quantités catalytiques d'acétate de diisopropylamine ou d'acétate de diméthylbenzylamine.

2. Procédé suivant la revendication 1, caractérisé en ce qu'on utilise comme solvants organiques des alcools aliphatiques ayant jusqu'à 6 atomes de carbone.

3. Procédé suivant la revendication 1, caractérisé en ce qu'on conduit la réaction entre -10 et 150 ° C.

4. Procédé suivant la revendication 1, caractérisé en ce qu'on conduit la réaction en présence de 0,001 à 0,009 mole de catalyseur, par rapport aux quantités molaires du composé ylidénique II.

5. Utilisation de l'acétate de diisopropylamine ou de l'acétate de diméthylbenzylamine comme catalyseur dans la production de la nitrendipine.